# EUROPEAN PATENT APPLICATION

(11) **EP 4 289 926 A1**
(43) Date of publication of application: **13.12.2023**
(21) Application number: 22177376.5
(22) Date of filing: 06.06.2022
(51) Int. Cl.: C12M 1/32, C12M 3/06, C12M 1/00

(54) **BIOLOGIC SCAFFOLD-ASSISTED CELL CULTIVATION**

(71) Applicant: Finnadvance Oy, 90220 Oulu (FI)
(72) Inventor: Singh, Prateek, 90220 Oulu (FI); Nguyen, Hoang Tuan, 90220 Oulu (FI)
(74) Representative: Papula Oy

(57) **Abstract**

The present disclosure relates to a cell culture apparatus provided with one or more biologic scaffolds (e.g., tissue-specific, bone-specific and/or organ-specific scaffolds), a method for cell cultivation by using the cell culture apparatus, and a cell culture incubator comprising the cell culture apparatus. More specifically, the cell culture apparatus comprises a plurality of cell culture modules arranged adjacent to each other. Each cell culture module comprises two or more culture medium reservoirs connected by two or more flow channels to a top-loaded chamber. The top-loaded chamber has a porous biologic scaffold arranged therein such that the biologic scaffold is at least partly exposed to a culture medium flow passing between the culture medium reservoirs through the top-loaded chamber. The apparatus thus configured may facilitate efficient biologic scaffold-assisted cell cultivation *in vitro.*

## Description

### TECHNICAL FIELD

The present disclosure relates generally to the field of biotechnology. In particular, the present disclosure relates to a cell culture apparatus provided with one or more biologic scaffolds (e.g., tissue-specific, bone-specific and/or organ-specific scaffolds), a method for cell cultivation by using the cell culture apparatus, and a cell culture incubator comprising the cell culture apparatus.

### BACKGROUND

Cell culture or cell cultivation involves growing cells of desired type(s) outside a living body under controlled *in vitro* conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Human tissue engineering is of particular interest due to the growing demand for human tissues and organs, which is caused by different diseases and/or accidents. In this regard, biologic scaffolds are usually used, on which cells are cultured to form tissues and/or organs of interest. There are two common methods for such tissue and/or organ preparation, namely: (1) the addition of biologic scaffolds, growth factors, and cells into a culture medium for *in vitro* organization; and (2) the implantation of biologic scaffolds and growth factors into a human body for *in vivo* organization. Thus, the first method involves artificial cell growth on the surfaces of the biologic scaffolds, while the second method involves natural cell growth on the surfaces of the biologic scaffolds.

However, there are a few challenges in the natural cell growth on the surfaces of the biologic scaffolds, namely: a lack of vascularized networks for transporting nutrients and waste and a lack of regulation of cell behaviors, such as the growth, proliferation, and differentiation of cells adhering to the biologic scaffolds. Therefore, the artificial cell growth on the surfaces of the biologic scaffolds is sometimes preferable. At the same time, more engineering is still required to promote the scaffold-assisted tissue and/or organ preparation *in vitro.*

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a technical solution that enables cell cultivation by using one or more biologic scaffolds (e.g., tissue-specific scaffolds, bone-specific scaffolds and/or organ-specific scaffolds).

The objective above is achieved by the features of the independent claims in the appended claims. Further embodiments and examples are apparent from the dependent claims, the detailed description and the accompanying drawings.

According to a first aspect, a cell culture apparatus is provided. The apparatus comprises a plurality of cell culture modules arranged adjacent to each other. Each cell culture module of the plurality of cell culture modules comprises at least two culture medium reservoirs, a top-loaded chamber, at least two flow channels, and a biologic scaffold. The top-loaded chamber is arranged between the at least two culture medium reservoirs and is defined by a bottom and a lateral surface. The lateral surface of the top-loaded chamber has at least two holes near the bottom. Each of the at least two flow channels connects one of the at least two culture medium reservoirs to one of the at least two holes of the lateral surface of the top-loaded chamber. The biologic scaffold has through pores formed therein. The biologic scaffold is arranged in the top-loaded chamber such that the biologic scaffold is at least partly exposed to a culture medium flow between the at least two culture medium reservoirs through the top-loaded chamber. The apparatus further comprises a flow driving unit configured to create the culture medium flow in each cell culture module of the plurality of cell culture modules. The apparatus thus configured may facilitate efficient biologic scaffold-assisted cell cultivation *in vitro.*

In one exemplary embodiment of the first aspect, the biologic scaffold is arranged at the bottom of the top-loaded chamber in at least one cell culture module of the plurality of cell culture modules. Due to this arrangement, the biologic scaffold may be much more exposed to the culture medium flow. For example, if the biologic scaffold is small enough (e.g., compared to the cross-sectional area of the culture medium flow), it will lie on the bottom of the top-loaded chamber, while being completely "covered" by the culture medium flow. If the biologic scaffold is large enough (e.g., compared to the to the cross-sectional area of the culture medium flow), some part of the biologic scaffold may rise above the culture medium flow. In both examples, different biologic scaffold-based cell systems may be obtained.

In one exemplary embodiment of the first aspect, the top-loaded chamber in at least one cell culture module of the plurality of cell culture modules is divided by the biologic scaffold into an apical sub-chamber and a basal sub-chamber. The apical sub-chamber and the basal sub-chamber are in fluid communication with each other via the through pores of the biologic scaffold, and the at least two holes are provided in the basal sub-chamber. In this embodiment, the biologic scaffold may serve as a thick porous septum between the apical and basal sub-chambers and may be used to mimic different biological barriers for different cell behavior studies.

In one exemplary embodiment of the first aspect, at least one cell culture module of the plurality of cell culture modules further comprises a porous membrane arranged in the top-loaded chamber such that the top-loaded chamber is divided by the porous membrane into an apical sub-chamber and a basal sub-chamber. The apical sub-chamber and the basal sub-chamber are in fluid communication with each other via the porous membrane, and the at least two holes are provided in the basal sub-chamber. The biologic scaffold is arranged on the porous membrane in the apical sub-chamber. In this embodiment, the porous membrane may serve as a thin porous septum between the apical and basal sub-chambers and may be used to mimic different biological barriers for different cell behavior studies. Furthermore, in this embodiment, it is possible to provide the coculture of cells deposited on the bottom surface of the porous membrane (due to the culture medium flow) and the biologic scaffold placed on the top surface of the porous membrane.

In one exemplary embodiment of the first aspect, the biologic scaffold in at least one cell culture module of the plurality of cell culture modules is configured as a tissue-specific scaffold, a bone-specific scaffold, or an organ-specific scaffold. By using these types of biologic scaffolds, it is possible to form human tissues, bones and/or organs of interest.

According to a second aspect, a cell culture incubator is provided. The cell culture incubator comprises the cell culture apparatus according to the first aspect, and a pipetting station configured to feed a culture medium to each of the at least two culture medium reservoirs in each cell culture module of the plurality of cell culture modules. In the incubator thus configured, it is possible to efficiently perform the biologic scaffold-assisted cell cultivation *in vitro.*

According to a third aspect, a method for cell cultivation by using the cell culture apparatus according to the first aspect is provided. The method starts with the step of providing a culture medium to one of the at least two medium reservoirs in each cell culture module of the plurality of cell culture modules. Then, the method proceeds to the step of causing, by the flow driving unit, the culture medium to flow from said one of the at least two culture medium reservoirs to the rest of the at least two culture medium reservoirs in each cell culture module of the plurality of cell culture modules for a predefined time period. The predefined time period is selected based on the culture medium. By so doing, it is possible to efficiently perform the biologic scaffold-assisted cell cultivation *in vitro.*

In one embodiment of the third aspect, the culture medium is caused to flow by applying a positive pressure to the at least two culture medium reservoirs in each cell culture module of the plurality of cell culture modules for the predefined time period. By so doing, it is possible to provide a proper culture medium flow in each cell culture module, thereby improving the biologic scaffold-assisted cell cultivation in each cell culture module.

In another embodiment of the third aspect, the culture medium is caused to flow by applying a positive pressure to said one of the at least two culture medium reservoirs in each cell culture module of the plurality of cell culture modules for the predefined time period, while capping the rest of the at least two culture medium reservoirs. By so doing, it is possible to provide a proper culture medium flow in each cell culture module, thereby improving the biologic scaffold-assisted cell cultivation in each cell culture module.

In yet another embodiment of the third aspect, the at least two culture medium reservoirs in each cell culture module of the plurality of cell culture modules comprises a first culture medium reservoir and a second culture medium reservoir. In this embodiment, the culture medium is caused to flow by:
- applying a first positive pressure to the first culture medium reservoir in each cell culture module of the plurality of cell culture modules for the predefined time interval, while capping the second culture medium reservoir; and
- applying a second positive pressure to the second culture medium reservoir in each cell culture module of the plurality of cell culture modules for the predefined time interval, while capping the first culture medium reservoir.

By so doing, it is possible to provide a proper culture medium flow in each cell culture module, thereby improving the biologic scaffold-assisted cell cultivation in each cell culture module.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1 shows a block diagram of a cell culture apparatus in accordance with one exemplary embodiment;
FIG. 2 shows different views of a cell culture module included in the cell culture apparatus shown in FIG. 1 in accordance with a first exemplary embodiment, namely: FIG. 2A shows a top view of the cell culture module, and FIG. 2B shows a sectional side view of the cell culture module (as taken along line A-A in FIG. 2A);
FIG. 3 shows a sectional side view of a cell culture module included in the cell culture apparatus shown in FIG. 1 in accordance with a second exemplary embodiment;
FIG. 4 shows a sectional side view of a cell culture module included in the cell culture apparatus shown in FIG. 1 in accordance with a third exemplary embodiment;
FIG. 5 shows a block diagram of a cell culture incubator in accordance with one exemplary embodiment; and
FIG. 6 shows a flowchart of a method for cell cultivation by using the cell culture apparatus shown in FIG. 1 in accordance with one exemplary embodiment.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, apparatuses and/or method disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above", "under", "apical", "basal", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first culture medium reservoir discussed below could be called a second culture medium reservoir, and vice versa, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

As used in the embodiments disclosed herein, a culture medium, also referred to as a growth medium, may refer to a liquid, suspension, or gel designed to support cellular growth in an artificial environment, i.e., *in vitro.* There are different types of culture media suitable for growing different types of cells. In general, the culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

In the embodiments disclosed herein, a biologic scaffold (or bioscaffold for short) may refer to a scaffold applied in bioengineering (e.g., tissue engineering). In general, the biologic scaffold may be considered as a structural support for cell attachment (e.g., for the purpose of subsequent tissue, bone or organ development). Different biomaterials and manufacturing methods contribute to the diversity of biologic scaffolds and are well-known in this technical field. For example, collagen, various proteoglycans, alginate-based substrates, and chitosan may be used in the production of biologic scaffolds. The manufacturing methods may, for example, include any of the following techniques: extrusion-based 3D bioprinting, inkjet 3D bioprinting, Ultra-Violet (UV)-assisted 3D bioprinting, electrospinning/electrowriting, soft lithography, etc. The biologic scaffold may also be inorganic in nature and fabricated from ceramic, glass or metal structures. Examples of such inorganic biologic scaffold include nickel or titanium scaffolds formed by sintering, as well as hydroxyapatite or calcium phosphate scaffolds formed by 3D printing, which may be used for bone and cartilage culture applications. Carbon derivatives like graphene, carbon nanotubes may also serve as the scaffold material. Finally, plastic (e.g., polyethylene terephthalate (PET), polystyrene (PS), polypropylene (PP), and others) and resin materials may be used for scaffold fabrication using injection molding or similar mass manufacturing techniques. The biologic scaffold may be shaped differently, depending on particular applications (e.g., depending on a tissue, bone or organ to be formed based on the biologic scaffold). As an example, the biologic scaffold may be configured as a sponge-like structure, a lattice-like structure, a fibrous structure, etc.

As used in the embodiments disclosed herein, a cell culture apparatus may refer to an apparatus having various wells (e.g., reservoirs, chambers, etc.) connected by microchannels in which fluids (i.e., culture media) will exhibit microfluidic behavior in their flow through the microchannels. Such a cell culture apparatus is also referred to as a microfluidic chip in this technical field. The microfluidic cell culture performed by the cell culture apparatus is generally related to cell culture, maintenance and perturbation in micro-scale fluid volumes. The reasons behind the popularity of the microfluidic cell culture are both economic and scientific. The microfluidic chips have the advantages of *in vitro* cell culture (high-throughput, parallel experiments, experiments may be done at the discretion of an experimenter, no need for specialized infrastructure and personnel, etc.) with *in vivo* like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

In the embodiments disclosed herein, each microchannel of the cell culture apparatus is also referred to as a flow channel and may relate to a sub-millimeter-scale channel that has a hydraulic diameter below 1 mm and is used for fluidically connecting the wells of the cell culture apparatus. In other words, the microchannel or flow channel generally denotes a channel configured to pass different fluids, such, for example, as culture media (e.g., cell suspensions), reagents, or gels, in micro-scale volumes. The microchannel may be shaped such that it has appropriate flow characteristics (e.g., flow rates) depending on particular applications. For example, the microchannel may have a rectangular, e.g., square, or rounded cross-section, as well as be straight, bended, or tilted towards a required direction.

The exemplary embodiments disclosed herein provide a technical solution that enables cell cultivation by using one or more biological scaffolds. More specifically, a cell culture apparatus is provided, which comprises a plurality of cell culture modules arranged adjacent to each other. Each cell culture module comprises two or more culture medium reservoirs connected by two or more flow channels to a top-loaded chamber. The top-loaded chamber has a porous biologic scaffold arranged therein such that the biologic scaffold is at least partly exposed to a culture medium flow passing between the culture medium reservoirs through the top-loaded chamber. The apparatus thus configured may facilitate efficient biologic scaffold-assisted cell cultivation *in vitro.*

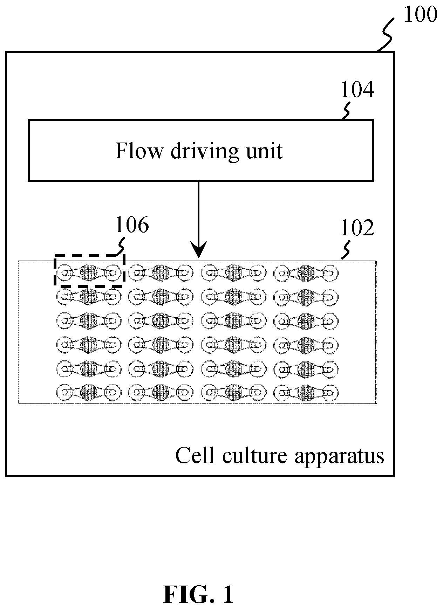

FIG. 1 shows a block diagram of a cell culture apparatus 100 in accordance with one exemplary embodiment. The apparatus 100 is intended for the above-mentioned microfluidic cell culture. As shown in FIG. 1, the apparatus 100 comprises a cell culture plate 102 and a flow driving unit 104 coupled to the cell culture plate 102. The cell culture plate 102 comprises multiple cell culture modules 106 and may be configured to fit the standard 96, 384, or 1536 microtiter plates which are well-known in this technical field. Each cell culture module 106 may be configured as one- or multi-layered structure made of roomtemperature-vulcanizing (RTV) silicone (e.g., RTV615) or polydimethylsiloxane (PDMS), and the cell culture modules 106 may be attached to each other in the order shown in FIG. 1 by using adhesive or plasma bonding to implement the cell culture plate 102. The flow driving unit 104 is configured to cause a culture medium to flow in each cell culture module 106, as will be described below in more detail. It should be noted that the number, arrangement, and interconnection of the constructive elements constituting the apparatus 100, which are shown in FIG. 1, are not intended to be any limitation of the present invention, but merely used to provide a general idea of how the constructive elements may be implemented within the apparatus 100. For example, the cell culture plate 102 may be replaced with two or more cell culture plates, and the flow driving unit 104 may be replaced with two or more flow driving units each configured to control a culture medium flow in one of the cell culture plates.

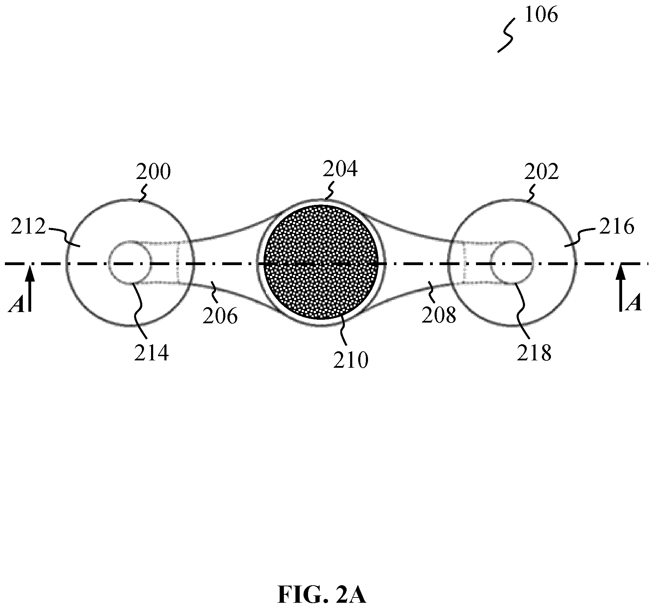

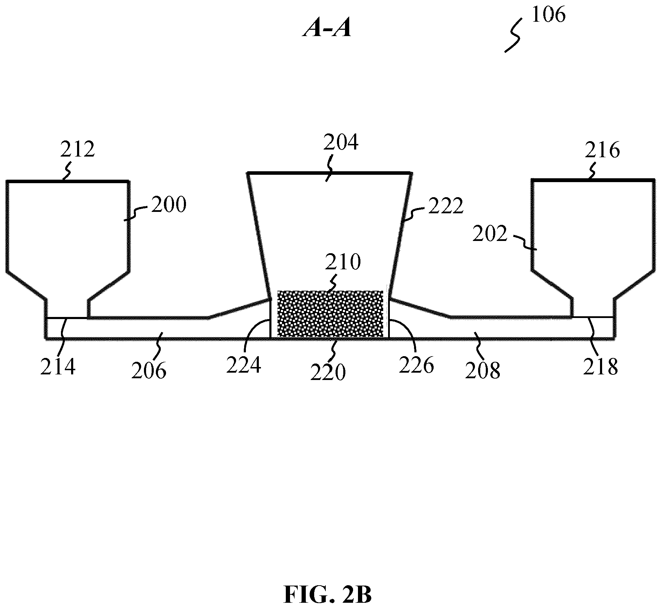

FIGs. 2A and 2B show different views of the cell culture module 106 included in the apparatus 100 in accordance with a first exemplary embodiment. More specifically, FIG. 2A shows a top view of the cell culture module 106, and FIG. 2B shows a sectional side view of the cell culture module 106 (as taken along line A-A in FIG. 2A). As shown in FIGs. 2A and 2B, the cell culture module 106 comprises a first culture medium reservoir 200, a second culture medium reservoir 202, a top-loaded (i.e., top-access) chamber 204, a first flow channel 206, a second flow channel 208, and a biological scaffold 210. It should be again noted that the present disclosure is not limited to the shown number, arrangement, and interconnection of the constructive elements of the cell culture module 106. In some embodiments, there may be more than two medium culture reservoirs, thereby leading to more than two flow channels. In other embodiments, there may be two or more top-loaded chambers between two or more culture reservoirs, and each of the culture medium reservoirs may be connected to each of the top-loaded chambers, thereby also increasing the number of the flow channels in the cell culture module 106.

The first culture medium reservoir 200 has a top part with an inlet 212 for a culture medium and a bottom part with an outlet 214 for the culture medium. Similarly, the second culture medium reservoir 202 has a top part with an inlet 216 for the culture medium and a bottom part with an outlet 218 for the culture medium. The first and second culture medium reservoirs 200 and 202 may be implemented as identical hollow tubes. Each of the first and second culture medium reservoirs 200 and 202 may have a circular, polygonal, oval or any other cross-section, if required and depending on particular applications. Moreover, the bottom part of each of the first and second culture medium reservoirs 200 and 202 may have a different profile, for example, a positively tapered profile, as shown in FIG. 2B.

The top-loaded chamber 204 is arranged between the first and second medium culture reservoirs 200 and 202. For example, the top-loaded chamber 204 may be aligned with the first and second culture medium reservoirs 200 and 202 in a horizontal direction. The top-loaded chamber 204 is defined by a bottom 220 and a lateral surface 222. The lateral surface 222 has two holes 224 and 226 near the bottom 220, as shown in FIG. 2B. The first chamber 204 is shaped as a hollow tube having a positively tapered circular profile. Again, the shown shape of the first chamber 204 is for illustrative purposes only and should not be considered as any limitation of the present disclosure. In some other embodiments, the first chamber 204 may be shaped as a hollow tube, for example, with a uniform circular or other (e.g., polygonal, oval, etc.) cross-section. As for the holes 224 and 226, they may be suitably shaped to achieve required flow characteristics (e.g., an appropriate flow rate). For example, the holes 224 and 226 may have a shape corresponding to the cross-section of the flow channels 206 and 208, respectively, at their connection to the top-loaded chamber 204.

The first and second flow channels 206 and 208 are microchannels providing the passage of the culture medium between the first and second culture medium reservoirs 200 and 202 through the top-loaded chamber 204. In particular, the first flow channel 206 connects the outlet 214 of the bottom part of the first culture medium reservoir 200 to the hole 224 arranged on the left side of the top-loaded chamber 204. The second flow channel 208 connects the outlet 218 of the bottom part of the second culture medium reservoir 202 to the hole 226 arranged on the right side of the top-loaded chamber 204. Each of the first and second flow channels 206 and 208 may have a longitudinal section and a cross-section which allow one to achieve required flow characteristics (e.g., an appropriate flow rate). For example, each of the first and second flow channels 206 and 208 may a variable channel height and a variable channel width which increase (e.g., gradually) towards the top-loaded chamber 204, as shown in FIGs. 2A and 2B.

In some embodiments, each of the first flow channel 206 and the second flow channel 208 may be at least partly bended or tilted relative to the horizontal direction such that the bottom 220 of the top-loaded chamber 204 is arranged higher than the bottom part of the first/second culture medium reservoir 200/202 (i.e., higher than the end of the outlet 214/218). For example, for each of the first flow channel 206 and the second flow channel 208, a tilting angle α relative to the horizontal direction may be within a range of 5 degrees to 45 degrees to provide appropriate flow characteristics.

The biologic scaffold 210 is arranged at the bottom 220 of the top-loaded chamber 204 in the cell culture module 106. The biologic scaffold 210 is assumed to have through pores that allow the culture medium flow to pass through the biologic scaffold 210. The through pores may have different cross-sectional shapes, such, for example, as triangular, rectangular, square, oval, or polygonal (e.g., hexagonal). The polygonal cross-section shape of the through pores may refer to a convex or concave polygon. In some other embodiments, the through pores may have different cross-sectional shapes, such as any combination of two or more of the above-mentioned cross-sectional shapes (e.g., the combination of circular and square cross-section shapes). Furthermore, each of the through pores may have a pore size varying within a predefined range of values. For example, the pore size may vary from 0.2 µm to 10 µm. In general, the predefined range of values for the pore size (as well as the spacing between the through pores) is selected based on certain cells comprised in the culture medium. The biologic scaffold 210 may be commercially available, or may be 3D printed in-place, or may be presented as a photopolymerid in-solution. The biologic scaffold 210 may be shaped such that it at least partly adjoins the lateral surface 222 (e.g., tightly adjoins the holes 224 and 226 of the lateral surface 222 of the top-loaded chamber 204). The biologic scaffold 210 may be sized to be fully covered by the culture medium flowing between the first and second culture medium reservoirs 200 and 202 through the top-loaded chamber 204. Alternatively, the biologic scaffold 210 may be sized such that it at least partly rises above the culture medium flow.

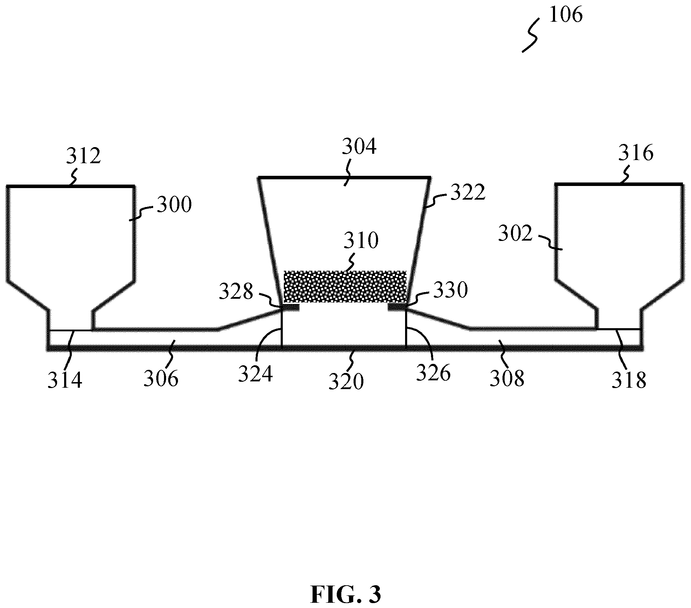

FIG. 3 shows a sectional side view of the cell culture module 106 in accordance with a second exemplary embodiment. The sectional side view shown in FIG. 3 is taken in the same manner as the sectional side view shown in FIG. 2B, i.e., by using similar section line A-A as in FIG. 2A. As shown in FIG. 3, the cell culture module 106 comprises a first culture medium reservoir 300, a second culture medium reservoir 302, a top-loaded (i.e., top-access) chamber 304, a first flow channel 306, a second flow channel 308, and a porous biological scaffold 310. The first culture medium reservoir 300 has a top part with an inlet 312 for a culture medium and a bottom part with an outlet 314 for the culture medium. Similarly, the second culture medium reservoir 302 has a top part with an inlet 316 for the culture medium and a bottom part with an outlet 318 for the culture medium. The top-loaded chamber 304 is arranged between the first and second medium culture reservoirs 300 and 302 and is defined by a bottom 320 and a lateral surface 322. The lateral surface 322 has two holes 324 and 326 near the bottom 320. In general, the first culture medium reservoir 300, the second culture medium reservoir 302, the first flow channel 306, and the second flow channel 308 may be implemented in the same or similar manner as the first culture medium reservoir 200, the second culture medium reservoir 202, the first flow channel 206, and the second flow channel 208, respectively.

The second embodiment shown in FIG. 3 differs from the first embodiment shown in FIGs. 2A and 2B in that the biologic scaffold 310 is arranged in the top-loaded chamber 304 with a gap from the bottom 320. In this case, the biologic scaffold 310 divides the top-loaded chamber 304 into an apical sub-chamber and a basal sub-chamber which are in fluid communication with each other via the through pores of the biologic scaffold 310. At this division, the holes 324 and 326 are in the basal sub-chamber. To provide such arrangement of the biologic scaffold 310, the top-loaded chamber 304 may have two support elements 328 and 330 provided on the lateral surface 322 inside the top-loaded chamber 304. Each of the support elements 328 and 330 may be sized to securely accommodate the biological scaffold 310 thereon, while allowing the biologic scaffold 310 to be exposed to the culture medium flow passing under the biologic scaffold 310. Instead of the support elements 328 and 330, a single support element may be used, which extends along the whole lateral surface 322 inside the top-loaded chamber 304 (e.g., such a single support element may be implemented as an inner ring). In other embodiments, the biologic scaffold 310 may be attached (e.g, somehow adhered) to the lateral surface 322 inside the top-loaded chamber 304. The biologic scaffold 310 may divide the top-loaded chamber 304 such that the basal sub-chamber has dimensions significantly smaller than the apical sub-chamber. For example, the apical sub-chamber may have a height of about 7 mm, while the height of the basal sub-chamber may fall within a range from 50 µm to 150 µm (at height values higher than 150 µm, the basal sub-chamber may lose microfluidic properties).

Furthermore, the biologic scaffold 310 may be made thinner than the biologic scaffold 210. In this case, it is possible to place, for example, one or more organoids on the top surface of the biologic scaffold 310 in order to study the interaction between the organoid(s) and the cells deposited from the culture medium flow on the bottom surface of the biologic scaffold 310.

It should be also noted that the tilted configuration of the flow channels 306 and 308 - i.e., when each of the flow channels 306 and 308 is tilted up at the above-mentioned angle α relative to the horizontal direction, - may be used to provide the cell deposition on the bottom surface of the biologic scaffold 310, without having to invert the cell culture module 106. In this case, the culture medium flow should have a flow rate greater than gravity and be continuous enough (e.g., lasts at least 30 min, preferably 60 min).

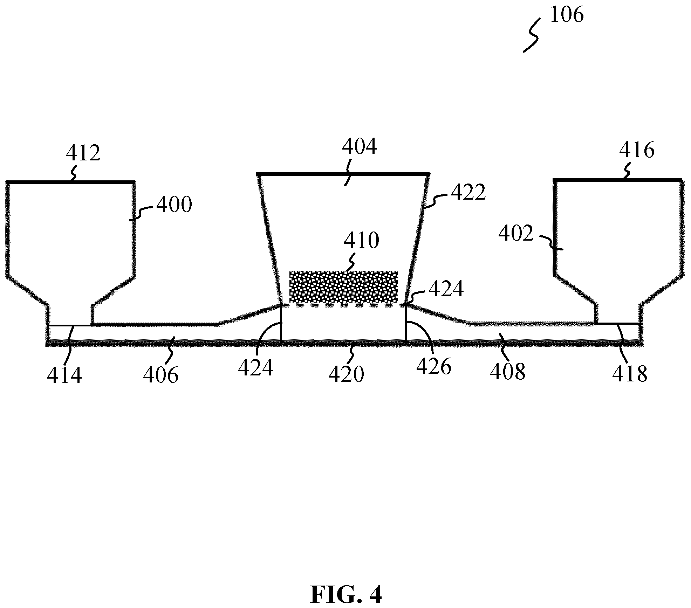

FIG. 4 shows a sectional side view of the cell culture module 106 in accordance with a third exemplary embodiment. The sectional side view shown in FIG. 4 is taken in the same manner as the sectional side view shown in FIG. 2B, i.e., by using similar section line A-A as in FIG. 2A. As shown in FIG. 4, the cell culture module 106 comprises a first culture medium reservoir 400, a second culture medium reservoir 402, a top-loaded (i.e., top-access) chamber 404, a first flow channel 406, a second flow channel 408, and a porous biological scaffold 410. The first culture medium reservoir 400 has a top part with an inlet 412 for a culture medium and a bottom part with an outlet 414 for the culture medium. Similarly, the second culture medium reservoir 402 has a top part with an inlet 416 for the culture medium and a bottom part with an outlet 418 for the culture medium. The top-loaded chamber 404 is arranged between the first and second medium culture reservoirs 400 and 402 and is defined by a bottom 420 and a lateral surface 422. The lateral surface 422 has two holes 424 and 426 near the bottom 420. In general, the first culture medium reservoir 400, the second culture medium reservoir 402, the first flow channel 406, and the second flow channel 408 may be implemented in the same or similar manner as the first culture medium reservoir 200, the second culture medium reservoir 202, the first flow channel 206, and the second flow channel 208, respectively.

The third embodiment shown in FIG. 4 differs from the first embodiment shown in FIGs. 2A and 2B in that the top-loaded chamber 404 has a porous membrane 424 arranged therein such that the top-loaded chamber 404 is divided by the porous membrane 424 into an apical sub-chamber and a basal sub-chamber. The apical sub-chamber and the basal sub-chamber are in fluid communication with each other via the porous membrane 424. In this case, the holes 424 and 426 are provided in the basal sub-chamber. The biologic scaffold 410 is arranged on the top surface of the porous membrane 424 in the apical sub-chamber. The porous membrane 424 may divide the top-loaded chamber 404 such that the basal sub-chamber has dimensions significantly smaller than the apical sub-chamber. For example, the apical sub-chamber may have a height of about 7 mm, while the height of the basal sub-chamber may fall within a range from 50 µm to 150 µm (at height values higher than 150 µm, the basal sub-chamber may lose microfluidic properties). The porous membrane 424 may be composed of silicone, plastic, protein, natural polymers, artificial polymers (e.g., polyester (PET)), metallic polymers or carbohydrates (e.g., cellulose), and may be formed by using one of the following methods: lithography, stamping, casting, electrospinning or *in situ* polymerization. The porous membrane 424 is assumed to have through pores configured such that the biologic scaffold 410 (i.e., its bottom surface) may be exposed to the culture medium flow passing through the basal sub-chamber of the top-loaded chamber 404. Again, the biologic scaffold 410 may be made thinner than the biologic scaffold 210, if required and depending on particular applications.

It should be again noted that the tilted configuration of the flow channels 406 and 408 - i.e., when each of the flow channels 406 and 408 is tilted up at the above-mentioned angle α relative to the horizontal direction, - may be used to provide the cell deposition on the bottom surface of the porous membrane 424, without having to invert the cell culture module 106. In this case, the culture medium flow should have a flow rate greater than gravity and be continuous enough (e.g., lasts at least 30 min, preferably 60 min).

It should be also noted that the cell culture plate 102 may comprise different cell culture modules 106 implemented in accordance with any two or more of the first, second and third embodiments discussed above. For example, one half of the cell culture modules 106 may be implemented in accordance with the first embodiment, while another half of the cell culture modules 106 may be implemented in accordance with the third embodiment. The cell culture plate 102 may also comprise the cell culture modules 106 implemented in accordance with each of the first, second and third embodiments.

Referring back to FIG. 1, the flow driving unit 104 is configured to cause the culture medium to flow, in each cell culture module 106 of the cell culture plate 102, between the first culture medium reservoir 200, 300 or 400 and the second culture medium reservoir 202, 302 or 402.

In one embodiment, the flow driving unit 104 may be a rocking platform that is configured to periodically rock the cell culture plate 102, thereby providing the culture medium flow. In another embodiment, the flow driving unit 104 may be a pneumatic pump that is configured to supply a compressed gas or a pressurized air to the inlet of each of the first and second culture medium reservoirs in each cell culture module 106 of the cell culture plate 102, thereby causing the culture medium flow therein. In yet another embodiment, the flow driving unit 104 may be configured to cause the culture medium flow in each cell culture module 106 of the cell culture plate 102 by pipetting the culture medium from the first culture medium reservoir 200, 300 or 400 to the second culture medium reservoir 202, 302 or 402, or vice versa, at regular time intervals or based on a level of the culture medium in each of the reservoirs.

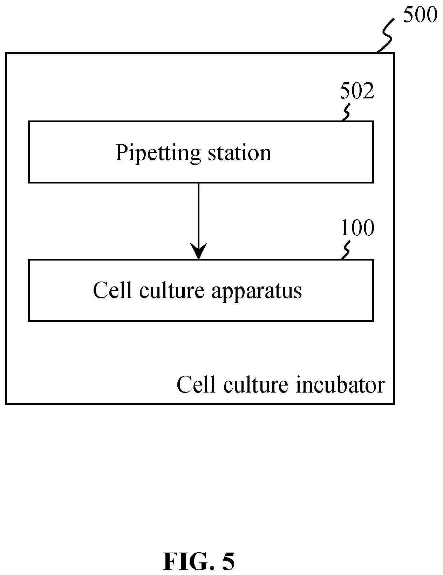

FIG. 5 shows a block diagram of a cell culture incubator 500 in accordance with one exemplary embodiment. The cell culture incubator 500 comprises the cell culture apparatus 100, and a pipetting station 502 coupled to the apparatus 500. More specifically, the pipetting station 502 is configured to feed the culture medium to each of the first culture medium reservoir 200, 300 or 400 and the second culture medium reservoir 202, 302 or 402 in each cell culture module 106 of the cell culture plate 102. The pipetting station 502 is well-known in this technical field, for which reason its description is omitted herein.

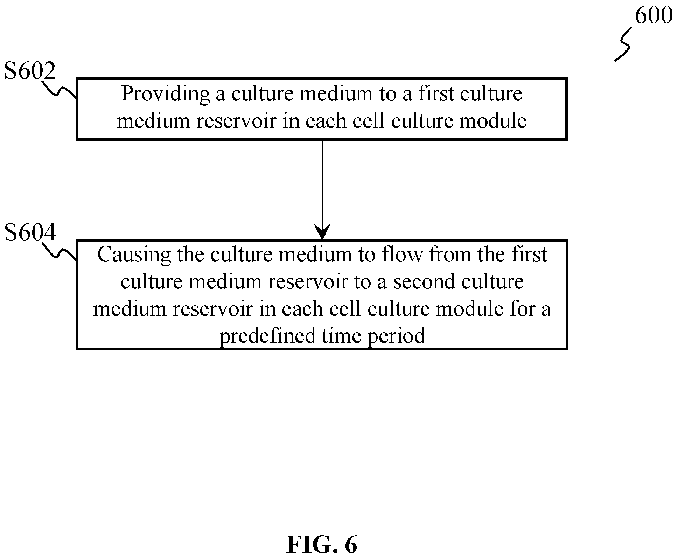

FIG. 6 shows a flowchart of a method 600 for cell cultivation by using the cell culture apparatus 100 in accordance with one exemplary embodiment. The method 600 starts with a step S602, in which a required culture medium is provided, e.g., the pipetting station 502, to one of the first culture medium reservoir 200, 300 or 400 and the second culture medium reservoir 202, 302 or 402 in each cell culture module 106 of the cell culture plate 102. Let us assume that the culture medium is provided to the first culture medium reservoir 200, 300 or 400. Then, the method 600 proceeds to a step S604, in which the flow driving unit 104 causes the culture medium to flow from the first culture medium reservoir 200, 300 or 400 to the second culture medium reservoir 202, 302 or 402, respectively, in each cell culture module 106 of the cell culture plate 102 for a predefined time period. The predefined time period may be selected based on the culture medium (i.e., a type of cells used therein).

In one embodiment, the step S604 of the method 600 may be performed by applying a positive pressure to each of the first culture medium reservoir 200, 300 or 400 and the second culture medium reservoir 202, 302 or 402 in each cell culture module 106 of the cell culture plate 102 for the predefined time period (e.g., 60 min). In another embodiment, the step S604 of the method 600 may be performed by applying a positive pressure to the first two culture medium reservoir 200, 300 or 400 in each cell culture module 106 of the cell culture plate 102 for the predefined time period (e.g., 60 min), while capping the second culture medium reservoir 202, 302 or 402, or vice versa. In yet another embodiment, the step S604 of the method 600 may be performed by: (i) applying a first positive pressure to the first culture medium reservoir 200, 300 or 400 in each cell culture module 106 of the cell culture plate 102 for the predefined time interval, while capping the second culture medium reservoir 202, 302 or 402; and (ii) applying a second positive pressure to the second culture medium reservoir 202, 302 or 402 in each cell culture module 106 of the cell culture plate 102 for the predefined time interval, while capping the first culture medium reservoir 200, 300 or 400. The selection of each of the above-indicated embodiments of the step S604 depends on particular applications.

Although the exemplary embodiments of the present disclosure are described herein, it should be noted that any various changes and modifications could be made in the embodiments of the present disclosure, without departing from the scope of legal protection which is defined by the appended claims. In the appended claims, the word "comprising" does not exclude other elements, steps or operations, and the indefinite article "a" or "an" does not exclude a plurality. The mere fact that certain measures are recited in mutually different dependent claims does not indicate that a combination of these measures cannot be used to advantage.

## Claims

1. A cell culture apparatus comprising:
a plurality of cell culture modules arranged adjacent to each other, each cell culture module of the plurality of cell culture modules comprising:
- at least two culture medium reservoirs;
- a top-loaded chamber arranged between the at least two culture medium reservoirs, the top-loaded chamber having a bottom and a lateral surface, the lateral surface having at least two holes near the bottom;
- at least two flow channels each connecting one of the at least two culture medium reservoirs to one of the at least two holes of the lateral surface of the top-loaded chamber; and
- a biologic scaffold having through pores formed therein, the biologic scaffold being arranged in the top-loaded chamber such that the biologic scaffold is at least partly exposed to a culture medium flow between the at least two culture medium reservoirs through the top-loaded chamber; and
a flow driving unit configured to create the culture medium flow in each cell culture module of the plurality of cell culture modules.

2. The apparatus of claim 1, wherein the biologic scaffold is arranged at the bottom of the top-loaded chamber in at least one cell culture module of the plurality of cell culture modules.

3. The apparatus of claim 1, wherein the top-loaded chamber in at least one cell culture module of the plurality of cell culture modules is divided by the biologic scaffold into an apical sub-chamber and a basal sub-chamber, the apical sub-chamber and the basal sub-chamber being in fluid communication with each other via the through pores of the biologic scaffold, and the at least two holes being provided in the basal sub-chamber.

4. The apparatus of claim 1, wherein at least one cell culture module of the plurality of cell culture modules further comprises a porous membrane arranged in the top-loaded chamber such that the top-loaded chamber is divided by the porous membrane into an apical sub-chamber and a basal sub-chamber, the apical sub-chamber and the basal sub-chamber being in fluid communication with each other via the porous membrane, the at least two holes being provided in the basal sub-chamber, and the biologic scaffold being arranged on the porous membrane in the apical sub-chamber.

5. The apparatus of any one of claims 1 to 4, wherein the biologic scaffold in at least one cell culture module of the plurality of cell culture modules is configured as a tissue-specific scaffold, a bone-specific scaffold, or an organ-specific scaffold.

6. A cell culture incubator comprising:
the cell culture apparatus according to any one of claims 1 to 5; and
a pipetting station configured to feed a culture medium to each of the at least two culture medium reservoirs in each cell culture module of the plurality of cell culture modules.

7. A method for cell cultivation by using the cell culture apparatus according to any one of claims 1 to 5, the method comprising:
(a) providing a culture medium to one of the at least two medium reservoirs in each cell culture module of the plurality of cell culture modules; and
(b) causing, by the flow driving unit, the culture medium to flow from said one of the at least two culture medium reservoirs to the rest of the at least two culture medium reservoirs in each cell culture module of the plurality of cell culture modules for a predefined time period, the predefined time period being selected based on the culture medium.

8. The method of claim 7, wherein step (b) comprises applying a positive pressure to the at least two culture medium reservoirs in each cell culture module of the plurality of cell culture modules for the predefined time period.

9. The method of claim 7, wherein step (b) comprises applying a positive pressure to said one of the at least two culture medium reservoirs in each cell culture module of the plurality of cell culture modules forthe predefined time period, while capping the rest of the at least two culture medium reservoirs.

10. The method of claim 7, wherein the at least two culture medium reservoirs in each cell culture module of the plurality of cell culture modules comprises a first culture medium reservoir and a second culture medium reservoir, and wherein step (b) comprises:
- applying a first positive pressure to the first culture medium reservoir in each cell culture module of the plurality of cell culture modules for the predefined time interval, while capping the second culture medium reservoir; and
- applying a second positive pressure to the second culture medium reservoir in each cell culture module of the plurality of cell culture modules for the predefined time interval, while capping the first culture medium reservoir.
